# EUROPEAN PATENT APPLICATION

(11) **EP 2 497 958 A2**
(43) Date of publication of application: **12.09.2012**
(21) Application number: 12001557.3
(22) Date of filing: 07.03.2012
(51) Int. Cl.: F15B 11/036

(54) **Fluid operated actuation system**

(30) Priority: 08.03.2011 US 201161450228 P
(71) Applicant: Norgren GmbH, 46519 Alpen (DE)
(72) Inventor: Riedel, Ralph, 47495 Rheinberg (DE); Gevers, Frank, 46395 Bocholt (DE)
(74) Representative: Hartwell, Ian Peter

(57) **Abstract**

A fluid operated actuation system (50) is provided. The fluid operated actuation system (50) comprises a fluid operated actuator (200) and a valve system (300). The fluid operated actuator (200) includes a housing (201) separated into a first portion (205) and at least a second portion (206). The fluid operated actuator (200) also includes a first piston (202) movable within the first portion (205) and separating the first portion (205) into a first fluid chamber (205a) and a second fluid chamber (205b). A second piston (203) is also provided that is movable within the second portion (206) and separating the second portion (206) into a third fluid chamber (206a) and a fourth fluid chamber (206b). The valve system (300) selectively provides fluid communication between a pressurized fluid source (211) and one or more of the fluid chambers (205a, 205b, 206a, 206b). The valve system (300) also selectively provides fluid communication between the second fluid chamber (205b) and the third fluid chamber (206a).

## Description

### TECHNICAL FIELD

The present invention relates to, fluid operated actuation systems, and more particularly, to a fluid operated actuation system including a fluid operated actuator with two or more pistons and a method of operating the fluid operated actuation system.

### BACKGROUND OF THE INVENTION

Fluid operated actuators convert a fluid pressure to a work piece using an actuator that typically consists of a piston in a cylinder. A piston rod coupled to the piston typically acts on the work piece to perform some task. Although there are various suitable fluids that may be used, the fluid applied to the actuator generally comprises pneumatic or hydraulic fluid, for example. Pneumatic operated actuators arc generally used where the compressibility of air is desired or to obtain much higher flow rates and thus faster response times while hydraulic operated actuators are generally employed when high actuating forces are required. Both fluids have advantages and in some situations, either pneumatic or hydraulic fluid may be used.

While using an actuator that operates under a constant force and/or speed throughout the actuator's range of motion is suitable for some applications, other situations may require an actuator that can deliver a first force during movement to a first position and a second force during movement to a second position. In some applications, the second force may be greater than the first force. In some applications, the second movement may be shorter than the first movement. One particular application is in a spot welding gun in which a pair of welding jaws must be brought together onto a work piece during a welding operation. The jaws clamp onto the work piece with a desired force. Therefore, at the end of a clamping motion, a relatively high actuation force is required by the welding jaws. However, an actuator that provides a high level of force typically provides a relatively small range of actuation travel and/or uses a significant amount of pressurized fluid. This can be a problem where the jaws of a spot welding gun must open wide in order to be positioned on the work piece. Therefore, a jaw actuator of the spot welding gun needs to move relatively rapidly during a first actuation span and a large force is not required. During the second actuation span, the jaws need to move only a small distance, but a larger clamping force is required. In other situations, the first actuation span and the second actuation span may be approximately equal.

Prior art actuators are known that include more than one actuation range with each actuation range having a different actuation force. However, many of these prior art systems operate using a combination of pneumatic and hydraulic fluids resulting in a complicated system. In other prior art actuators with more than one piston, each piston receives a pressurized fluid substantially independently.

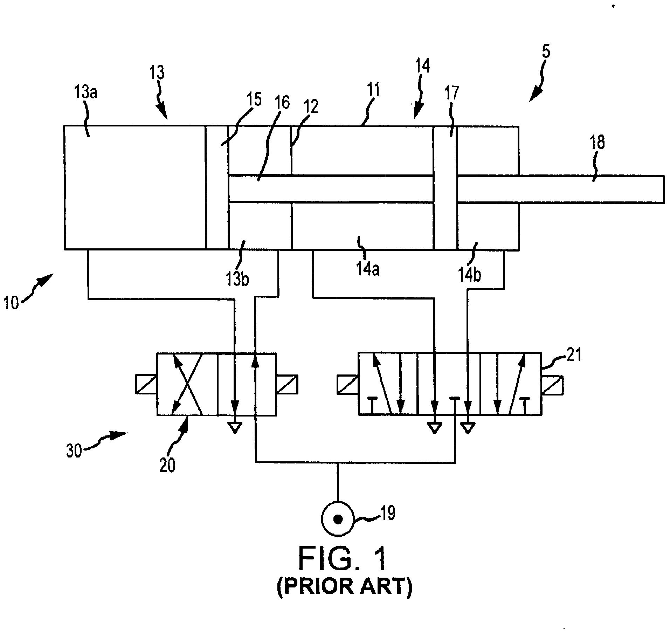

FIG. 1 shows a schematic of a prior art fluid operated actuation system 5. The fluid operated actuation system 5 comprises a fluid operated actuator 10 and a valve system 30. The prior art fluid operated actuator 10 includes a housing 11 separated by a wall 12. The wall 12 separates the housing 11 into a first portion 13 and a second portion 14. The first portion 13 comprises a first piston 15 and a first piston rod 16 coupled to and extending from the first piston 15. The piston rod 16 extends through the wall 12 and is coupled to a second piston 17. The second piston 17 includes a second piston rod 18 that is coupled to the second piston 17 and extends through the housing 11. As can be appreciated, each piston 15, 17 separates their respective portion of the housing 13, 14 into two chambers 13a, 13b, and 14a, 14b, respectively.

As can be appreciated, pressurization of chamber 13a, 14a, or both will cause the pistons 15, 17 and the piston rods 16, 18 to move to the right and thus, extend the piston rod 18 further from the housing 11. Conversely, pressurization of chamber 13b, 14b, or both will cause the pistons 15, 17 and the piston rods 16, 18 to move to the left and thus, retract the piston rod 18 into the housing 11. As can be seen, each of the portions 13, 14 of the housing 11 are in fluid communication with either a pressurized fluid supply 19 or an exhaust via first and second valves 20, 21. This type of configuration results in a substantial waste of pressurized fluid as the fluid operated actuator 10 is actuated to its various positions.

Operation of the prior art fluid operated actuation system 5 begins with actuating the first valve 20 of the valve system 30 to a first position and actuating the second valve 21 to a neutral position. The valves 20, 21 may be actuated electrically, pneumatically, mechanically, etc. When the first valve 20 is in the first position, the first chamber 13a is in fluid communication with the pressurized fluid source 19 while the second chamber 13b is exhausted. When the second valve 21 is in the neutral position, both fluid chambers 14a, 14b are open to exhaust to minimize any pressure buildup that may inhibit movement of the piston assembly. During the first stage of actuation, the piston assembly is moved only due to the pressure buildup in the first chamber 13a, which acts on the first piston 15. Consequently, the piston rod 18 acts on a work piece (not shown) with a first force.

Once the piston assembly reaches a predetermined position, the operator may want the piston rod 18 to act on the work piece with a second force that is greater than the first force. In order to increase the actuating force provided to the piston rod 18, the second valve 21 can be actuated to a first position where the first fluid chamber 14a is in fluid communication with the pressurized fluid source 19. Conversely, the second fluid chamber 14b is exhausted. Therefore, both of the first chambers 13 a, 14a are pressurized to approximately the same pressure, with the pressurized fluid acting on the first and second pistons 15, 17 to move the piston rod 18 to the right with a second force.

In order to retract the piston rod, one or both of the fluid chambers 13b, 14b can be brought into fluid communication with the pressurized fluid source while both of the first chambers 13a, 14a are exhausted.

While the prior art fluid operated actuation system 5 can actuate the piston rod 18 with a first force during a first actuation span and a second force during a second actuation span, the fluid operated actuation system 5 wastes a substantial amount of pressurized fluid, resulting in increased costs associated with operating the fluid operated actuation system 5. During extension of the piston rod 18 with the first force, pressurized fluid in the second fluid chamber 13b is exhausted. Likewise, pressurized fluid within the first and second chambers 14a, 14b is also exhausted. Consequently, when the piston rod 18 is actuated with the second force, the volume of the first chamber 14a must first be filled with pressurized fluid and once the pressure in the first chamber 14a reaches a threshold level, then the piston rod 18 is actuated with the second force. However, depending on the particular pressures used, a substantial amount of pressurized fluid may be required to fill the first chamber 14a prior to being able to provide the second force.

The present invention overcomes these and other problems and an advance in the art is achieved. The present invention provides a fluid operated actuation system including a fluid operated actuator with multiple pistons and controlled by a valve system. The present invention selectively provides a fluid communication path between two or more of the fluid chambers of the fluid operated actuator in order to minimize the amount of pressurized fluid that is wasted and exhausted from the system. Therefore, the present invention can reduce the operating costs associated with actuating the fluid operated actuator.

### SUMMARY OF THE INVENTION

A fluid operated actuation system is provided according to an embodiment of the invention. The fluid operated actuation system can include a fluid operated actuator with a housing separated into a first portion and at least a second portion. According to an embodiment of the invention, the fluid operated actuator further comprises a first piston movable within the first portion and separating the first portion into a first fluid chamber and a second fluid chamber. The fluid operated actuator can further include a second piston movable within the second portion and separating the second portion into a third fluid chamber and a fourth fluid chamber. According to an embodiment of the invention, the fluid operated actuation system further comprises a valve system selectively providing fluid communication between a pressurized fluid source and one or more of the fluid chambers and selectively providing fluid communication between the second fluid chamber and the third fluid chamber.

A method of operating a fluid operated actuation system is provided according to an embodiment of the invention. According to an embodiment of the invention, the fluid operated actuation system includes a fluid operated actuator with a housing separated into a first portion and a second portion. A first piston is provided that is movable in the first portion and separates the first portion into a first fluid chamber and a second fluid chamber. The fluid operated actuator further includes a second piston movable in the second portion and separating the second portion into a third fluid chamber and a fourth fluid chamber. According to an embodiment of the invention, a first piston rod is coupled to the first piston and extends from the first piston to contact the second piston. According to an embodiment of the invention, the method comprises steps of pressurizing the first fluid chamber and exhausting the fourth fluid chamber. The method further comprises a step of opening a fluid communication path between the second fluid chamber and the third fluid chamber to actuate the first and second pistons from a first position to a second position with a first actuation force.

### ASPECTS

According to an aspect of the invention, a fluid operated actuation system comprises:
a fluid operated actuator including:
   a housing separated into a first portion and at least a second portion;
   a first piston movable within the first portion and separating the first portion into a first fluid chamber and a second fluid chamber;
   a second piston movable within the second portion and separating the second portion into a third fluid chamber and a fourth fluid chamber; and
a valve system selectively providing fluid communication between a pressurized fluid source and one or more of the fluid chambers and selectively providing fluid communication between the second fluid chamber and the third fluid chamber.

Preferably, the valve system comprises a first valve that selectively provides fluid communication between the pressurized fluid source and the fourth fluid chamber in a first position and selectively provides fluid communication between the pressurized fluid source and the first fluid chamber in a second position.

Preferably, the valve system further comprises a second valve that selectively provides fluid communication between the first valve and the third fluid chamber.

Preferably, the valve system further comprises a third valve that selectively provides fluid communication between the second and third fluid chambers in a first position and exhausts the second fluid chamber in a second position.

Preferably, the fluid operated actuation system further comprises a separating member that separates the first and second portions of the housing.

Preferably, the fluid operated actuation system further comprises a first piston rod coupled to the first piston and extending through the separating member.

Preferably, the first piston rod is further coupled to the second piston.

Preferably, the fluid operated actuation system further comprises a second piston rod coupled to the second piston and extending through the housing.

According to another aspect of the invention, a method of operating a fluid operated actuation system including a fluid operated actuator with a housing separated into a first portion and a second portion, a first piston movable in the first portion and separating the first portion into a first fluid chamber and a second fluid chamber, a second piston movable in the second portion and separating the second portion into a third fluid chamber and a fourth fluid chamber, and a first piston rod coupled to the first piston and extending from the first piston to contact the second piston comprises steps of:
pressurizing the first fluid chamber;
exhausting the fourth fluid chamber; and
opening a fluid communication path between the second fluid chamber and the third fluid chamber to actuate the first and second pistons from a first position to a second position with a first actuation force.

Preferably, the steps of pressurizing the first fluid chamber and exhausting the fourth fluid chamber comprises actuating a first valve from a first position to a second position.

Preferably, the step of opening the fluid communication path between the second and third fluid chambers comprises actuating a second valve and a third valve to a first position.

Preferably, the method further comprises steps of:
closing the fluid communication path between the second and third fluid chambers;
exhausting the second fluid chamber; and
pressurizing the third fluid chamber to actuate the first and second pistons from the second position to a third position with a second actuation force.

Preferably, the steps of closing the fluid communication path between the second and third fluid chambers and exhausting the second fluid chamber comprises actuating a third valve to a second position.

Preferably, the step of pressurizing the third fluid chamber comprises actuating a second valve to a second position.

### BRIEF DESCRIPTION OF THE DRAWINGS

FIG. 1 shows a schematic of a prior art fluid operated actuation system.
FIG. 2 shows a schematic of a fluid operated actuation system according to an embodiment of the invention.
FIG. 3 shows a schematic of the fluid operated actuation system according to another embodiment of the invention.
FIG. 4 shows a schematic of the fluid operated actuation system according to another embodiment of the invention.

### DETAILED DESCRIPTION OF THE INVENTION

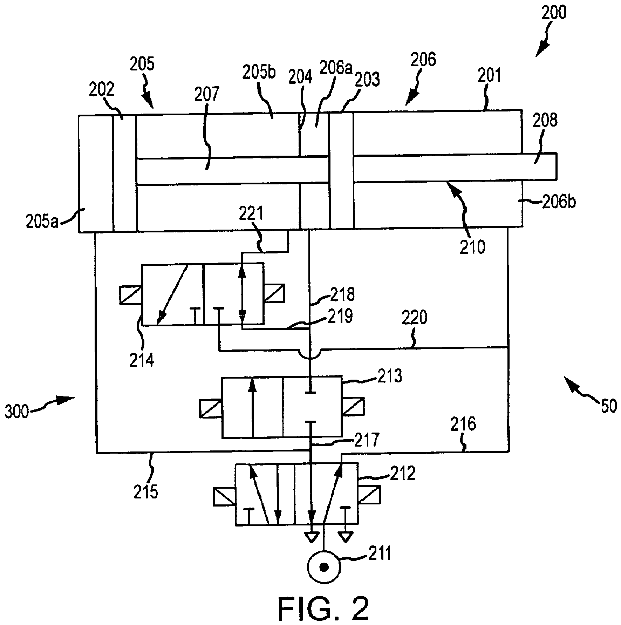

FIGS. 2 - 4 and the following description depict specific examples to teach those skilled in the art how to make and use the best mode of the invention. For the purpose of teaching inventive principles, some conventional aspects have been simplified or omitted. Those skilled in the art will appreciate variations from these examples that fall within the scope of the invention. Those skilled in the art will appreciate that the features described below can be combined in various ways to form multiple variations of the invention. As a result, the invention is not limited to the specific examples described below, but only by the claims and their equivalents.

**FIG. 2** shows a schematic of a fluid operated actuation system 50 according to an embodiment of the invention. The fluid operated actuation system 50 comprises a fluid operated actuator 200 and a valve system 300. According to an embodiment of the invention, the fluid operated actuator 200 comprises a housing 201 and two or more pistons 202, 203 movably located within the housing 201. Although the embodiment shown in the figures and described below includes two pistons 202, 203, it should be appreciated that the present invention should not be limited to two pistons, and in some embodiments, the fluid operated actuator 200 may include more than two pistons. According to an embodiment of the invention, the fluid operated actuator 200 further includes a separating member 204. The separating member 204 may comprise a membrane, a wall, or some other type of sealing member that can separate the housing 201 into a first portion 205 and a second portion 206. In some embodiments, the housing 201 may be separated into two or more sections that can be coupled where each section can house one or more portions of the fluid operated actuator 200.

According to an embodiment of the invention, the first piston 202 is movable within the first portion 205 of the housing 201. As shown in the figures, the first piston 202 can separate the first portion 205 into a first fluid chamber 205a and a second fluid chamber 205b. According to an embodiment of the invention, the second piston 203 is movable within the second portion 206 of the housing 201. The second piston 203 can separate the second portion 206 into a third fluid chamber 206a and a fourth fluid chamber 206b.

According to an embodiment of the invention, the fluid operated actuator 200 can include a first piston rod 207 that can be coupled to the first piston 202. Therefore, the first piston rod 207 is also movable within the first portion 205 of the housing 201. The first piston rod 207 can extend through the separating member 204 to contact the second piston 203. In some embodiments, the first piston rod 207 may be coupled to the second piston 203; however, in other embodiments, the first piston rod 207 can simply contact the second piston 203.

According to an embodiment of the invention, the fluid operated actuator 200 can also include a second piston rod 208. The second piston rod 208 can be coupled to the second piston 203. Therefore, the second piston rod 208 can be movable within the second portion 206 of the housing 201. The second piston rod 208 may extend through the housing 201 as shown. In the embodiment shown, the second piston rod 208 extends through an end of the housing 201. The second piston rod 208 can contact a work piece (not shown) to perform some type of work as is generally known in the art.

According to an embodiment of the invention, the first and second pistons 202, 203 and the first and second piston rods 207, 208 may be referred to collectively as a piston assembly 210. As explained in more detail below, the piston assembly 210 can move as a single unit within the housing 201. In one embodiment, the unitary movement of the piston assembly 210 is made possible by the first piston 207, which is coupled to the first piston 202 and in contact with or coupled to the second piston 203. Although not shown in the schematic provided in the figures, those skilled in the art will readily appreciate that the fluid operated actuator 200 will also include suitable seals to provide a fluid-tight seal between the pistons 202, 203 and the housing 201 as well as between the first piston rod 207 and the separating member 204 and the second piston rod 208 and the housing 201 as is generally known in the art. The particular type of seals used may depend upon the particular application and should in no way limit the scope of the present invention.

According to an embodiment of the invention, the piston assembly 210 can be actuated using the valve system 300, which is in fluid communication with the fluid operated actuator 200 to selectively provide a fluid communication path between one or more desired fluid chambers 205a, 205b, 206a, 206b and a pressurized fluid source 211. The pressurized fluid source 211 may comprise any suitable pressurized fluid, such as pneumatic or hydraulic, for example. According to an embodiment of the invention, the valve system 300 can also be configured to selectively provide fluid communication between the second and third fluid chambers 205b, 206a as explained in more detail below. According to an embodiment of the invention, the valve system 300 comprises a first valve 212, a second valve 213, a third valve 214, and a plurality of fluid lines 215-221. It should be understood that the particular valve arrangement described is merely one example. Those skilled in the art will readily recognize suitable alternative valve arrangements that achieve the same operation. Therefore, the present invention should not be limited to the particular valve arrangement shown and described.

According to an embodiment of the invention, the first valve 212 is in fluid communication with the pressurized fluid source 211 as well as a first fluid line 215 and a second fluid line 216. The first fluid line 215 is coupled to the first fluid chamber 205a while the second fluid line 216 is coupled to the fourth fluid chamber 206b. Advantageously, the first valve 212 can selectively provide fluid communication between the pressurized fluid source 211 and the first fluid chamber 205a or the fourth fluid chamber 206b. According to an embodiment of the invention, the first valve 212 comprises a 5/2-way valve. However, other configurations are contemplated, such as a 5/3-way valve that can close off both the first fluid chamber 205a and the fourth fluid chamber 206b from the pressurized fluid source 211. Further, while the first valve 212 is shown as comprising an electrically actuated valve (solenoid, piezo, shape memory alloy, etc.), other types of valves and configurations are contemplated. Therefore, the particular type of valve shown should in no way limit the scope of the present invention and those skilled in the art will readily recognize suitable alternatives.

According to an embodiment of the invention, when the first valve 212 is actuated to a first position, the first fluid chamber 205a is in fluid communication with an exhaust and the fourth fluid chamber 206b is in fluid communication with the pressurized fluid source 211. Conversely, if the first valve 212 is actuated to a second position, the fourth fluid chamber 206b is in fluid communication with the exhaust and the first fluid chamber 205a is in fluid communication with the pressurized fluid source 211.

According to an embodiment of the invention, the valve system 300 also includes a second valve 213 that is in fluid communication with the first valve 212 via the first fluid line 215 and a third fluid line 217, which branches off from the first fluid line 215. According to an embodiment of the invention, the second valve 213 is also coupled to a fourth fluid line 218, which is further coupled to the third fluid chamber 206a of the fluid operated actuator 200. According to an embodiment of the invention, when the second valve 213 is actuated to a first position, the third fluid chamber 206a is closed off from the first valve 212. Conversely, when the second valve 213 is actuated to a second position, the second valve 213 provides a fluid communication path between the first valve 212 and the third fluid chamber 206a. While the second valve 213 is shown as comprising a 2/2-way electrically actuated valve, those skilled in the art will readily recognize suitable alternatives. Therefore, the present invention should in no way be limited to a 2/2-way valve.

According to an embodiment of the invention, the valve system 300 further comprises a third valve 214. The third valve 214 is coupled to a fifth fluid line 219 that is further coupled to and branches off from the fourth fluid line 218. According to an embodiment of the invention, a sixth fluid line 220 is also coupled to the third valve 214. The sixth fluid line 220 provides a fluid communication path between the third valve 214 and the second fluid line 216. A seventh fluid line 221 is also coupled to the third valve 214. The seventh fluid line 221 is further coupled to the second fluid chamber 205b to provide a fluid communication path between the third valve 214 and the second fluid chamber 205b. Although the third valve 214 is shown as a 3/2-way electrically actuated valve, other configurations are certainly possible and the present invention should not be limited to the specific valve configuration shown. As shown in the figures, when the third valve 214 is actuated to a first position, the second fluid chamber 205b is selectively brought into fluid communication with the third fluid chamber 206a. According to an embodiment of the invention, when the third valve 214 is actuated to a second position, the second fluid chamber 205b is exhausted and the second and third fluid chambers 205b, 206a are closed off from one another.

Operation of the fluid operated actuation system 50 will now be described according to an embodiment of the invention. FIG. 2 shows the fluid operated actuator 200 with the piston assembly 210 in a retracted position. In order to move the piston assembly 210 to the left, *i.e.,* retract the piston assembly 210 at least partially into the housing 201 as shown in FIG. 2, the valves 212, 213, 214 can be actuated to a first position as shown in FIG. 2. With the first valve 212 in the first position, the fourth fluid chamber 206b is selectively brought into fluid communication with the pressurized fluid source 211 while the first fluid chamber 205a is exhausted. Furthermore, with the second valve 213 closed and the third valve 214 actuated to the first position, the second and third fluid chambers 205b, 206a are selectively brought into fluid communication with one another while being closed off from the pressurized fluid source 211 or an exhaust. Therefore, as the pressurized fluid provided to the fourth fluid chamber 206b biases the piston assembly 210 to the left, the volume of the third fluid chamber 206a decreases and the volume of the second fluid chamber 205b increases. This change in volumes creates a pressure differential between the second and third fluid chambers 205b, 206a causing pressurized fluid in the third fluid chamber 206a to exhaust into the second fluid chamber 205b. Therefore, rather than simply exhausting the third fluid chamber 206a and losing the pressurized fluid as in the prior art, the pressurized fluid in the third fluid chamber 206a can be transferred to the second fluid chamber 206b for later use as described below. In some embodiments, the transfer of the pressurized fluid may also provide an increased biasing force on the piston assembly 210 by acting on the first piston 202 in the second fluid chamber 205b. However, in other embodiments, the increased fluid in the second fluid chamber 205b may simply fill the increased volume to prevent a vacuum from forming in the second fluid chamber 205b that could resist movement of the piston assembly 210.

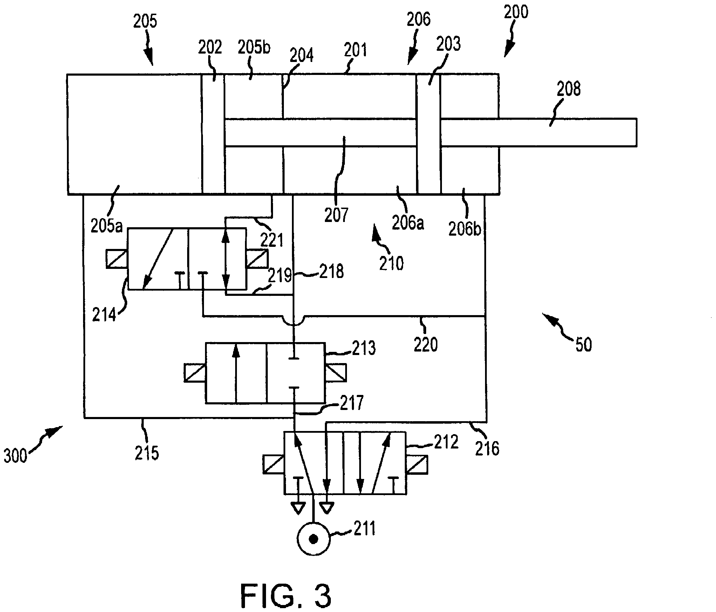

**FIG. 3** shows a schematic of the fluid operated actuation system 50 according to another embodiment of the invention. In FIG. 3, the piston assembly 210 has moved to the right from the position shown in FIG. 2 with a first force. In order to move the piston assembly 210 from the position shown in FIG.2 to a second position to the right (FIG. 3), *i.e.,* extend the piston rod 208 further from the housing 201, the first valve 212 can be actuated from the first position to a second position as shown in FIG. 3, while the second and third valves 213, 214 can remain in their first position. As can be appreciated, with the first valve 212 actuated to the second position, the first fluid chamber 205a is brought into fluid communication with the pressurized fluid source 211 while the fourth fluid chamber 206b is exhausted. With the second and third valves 213, 214 remaining in the first position, the second and third fluid chambers 205b, 206a are still in fluid communication with one another. However, with pressurized fluid being supplied to the first fluid chamber 205a, the piston assembly 210 is biased to the right thereby causing a decrease in the volume of the second fluid chamber 205b and an increase in the volume of the third fluid chamber 206a. Therefore, pressurized fluid is transferred from the second fluid chamber 205b to the third fluid chamber 206a due to the pressure differential between the two fluid chambers 205b, 206a. Advantageously, the pressurized fluid in the second fluid chamber 205b is not wasted to the environment during this stage. Rather, the third fluid chamber 206a is pressurized to a first pressure by the pressurized fluid from the second fluid chamber 205b. According to an embodiment of the invention, the decrease in volume of the second fluid chamber 205b is substantially equal to the increase in volume of the third fluid chamber 206a. Therefore, the transfer of pressurized fluid between the two chambers 205b, 206a prevents backpressure in the second fluid chamber 205b or a vacuum in the third fluid chamber 206a from countering movement of the piston assembly 210.

According to an embodiment of the invention, FIG. 3 shows the fluid operated actuation system 50 as the piston rod 208 acts on a work piece (not shown) with a first force during a first actuation span. During this stage, the first fluid chamber 205a is the only fluid chamber in fluid communication with the pressurized fluid source 211. Consequently, although some pressurized fluid is entering the third fluid chamber 206a from the second fluid chamber 205b, the pressurized fluid in the first fluid chamber 205a acting on the first piston 202 provides the majority, if not all, of the biasing force to the piston assembly 210. In other words, in some embodiments, the first pressure in the third fluid chamber 206a provided by pressurized fluid from the second fluid chamber 205b is not sufficient to increase the biasing force on the piston assembly 210 to the second force.

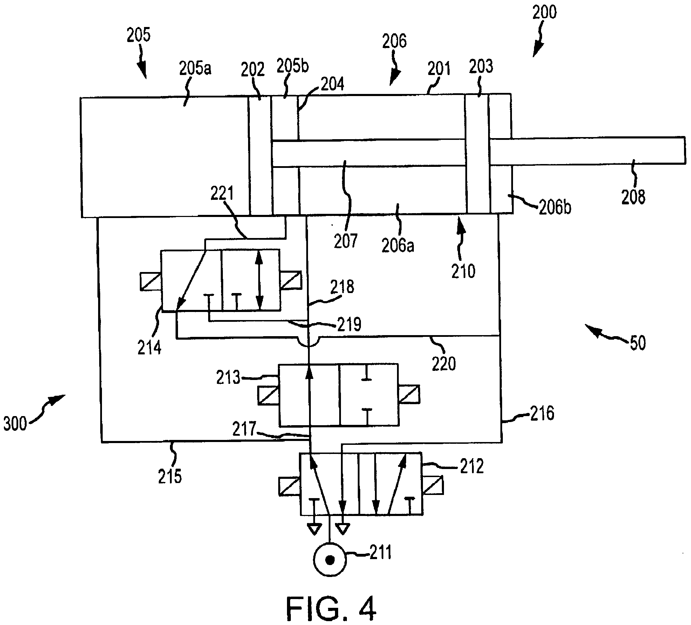

**FIG. 4** shows a schematic of the fluid operated actuation system 50 according to another embodiment of the invention. In FIG. 4, the second and third valves 213, 214 have been actuated from their first positions to their second positions while the first valve 212 has remained in the second position. In the second position, the first and second valves 212, 213 selectively provide fluid communication between the pressurized fluid source 211 and the third fluid chamber 206a. Because at least some pressurized fluid is already in the third fluid chamber 206a, a substantially smaller amount of pressurized fluid is required by the third fluid chamber 206a in order to increase the pressure in the third fluid chamber 206a to a desired operating pressure. Consequently, the reaction time to change from actuating the piston assembly 210 with the first force (FIG. 3) to actuating the piston assembly 210 with the second force (FIG. 4) is substantially less than in the prior art. Furthermore, actuation of the piston assembly 210 with the second force requires much less pressurized fluid, thereby reducing costs associated with actuating the piston assembly 210 with the second force.

In addition, with the third valve 214 in the second position, the fluid communication between the second and third fluid chambers 205b, 206a is closed off. Furthermore, the third valve 214 prevents fluid communication between the pressurized fluid source and the second fluid chamber 205b. Rather, the third valve 214 exhausts the second fluid chamber 205b. Therefore, FIG. 4 shows the fluid operated actuator 200 in the position where the piston assembly 210 is being extended with the second force acting on the piston assembly 210 that is greater than the first force. This is because, pressurized fluid from the pressurized fluid source 211 is now being supplied to both the first fluid chamber 205a and the third fluid chamber 206a to act on both the first piston 202 and the second piston 203, respectively. If the cross-sectional areas between the first and second pistons 202, 203 are substantially equal, the second force provided when both the first and third fluid chambers 205a, 206a are in fluid communication with the pressurized fluid source 211 is approximately double the first force when only the first fluid chamber 205a is in fluid communication with the pressurized fluid source. This approximation neglects the loss of cross-sectional area of the second piston 203 exposed to the pressurized fluid due to the first piston rod 207 contacting or coupling the second piston 203. Further, backpressure in the second and fourth fluid chambers 205b, 206b is minimized because both of these fluid chambers 205b, 206b are open to exhaust.

It should be appreciated that while the fluid operated actuator 200 is shown with two pistons 202. 203, in other embodiments, the fluid operated actuator 200 can include more than two pistons. In embodiments with more than two pistons 202, 203, the piston assembly 210 may be actuated with more than two forces over more than two actuation spans. In addition, while the present invention describes using the second force during an extension of the piston assembly 210, the present invention could be used rather to increase the force during retraction of the piston assembly 210 by simply rearranging the configuration of the third valve 214, for example. Therefore, the use of the terms first direction and second direction should not be limited to implying retraction and extension of the piston assembly 210.

The present invention as described above provides an improved fluid operated actuation system 50 capable of operating with a reduced pressurized fluid demand. By providing a valve system 300 that can selectively provide a fluid communication path between a pressurized fluid source 211 and one or more of the desired fluid chambers 205a, 205b, 206a, 206b the valve system 300 can actuate the piston assembly 210 over one or more actuation spans with one or more actuation forces. Furthermore, because the valve system 300 can additionally provide a fluid communication path between the second and third fluid chambers 205b, 206a, the third fluid chamber 206a can be pressurized to a first pressure when the piston assembly 210 is being actuated with the first force. Consequently, much less pressurized fluid is required to increase the pressure in the third fluid chamber 206a to a second pressure to actuate the piston assembly 210 with the second force. In addition, at least some of the pressurized fluid in the third fluid chamber 206a can be transferred to the second fluid chamber 205b during retraction in order to store the pressurized fluid for a later actuation.

The detailed descriptions of the above embodiments are not exhaustive descriptions of all embodiments contemplated by the inventors to be within the scope of the invention. Indeed, persons skilled in the art will recognize that certain elements of the above-described embodiments may variously be combined or eliminated to create further embodiments, and such further embodiments fall within the scope and teachings of the invention. It will also be apparent to those of ordinary skill in the art that the above-described embodiments may be combined in whole or in part to create additional embodiments within the scope and teachings of the invention.

Thus, although specific embodiments of, and examples for, the invention are described herein for illustrative purposes, various equivalent modifications are possible within the scope of the invention, as those skilled in the relevant art will recognize. The teachings provided herein can be applied to other fluid operated actuation systems, and not just to the embodiments described above and shown in the accompanying figures. Accordingly, the scope of the invention should be determined from the following claims.

## Claims

1. A fluid operated actuation system (50), comprising:
a fluid operated actuator (200) including:
a housing (201) separated into a first portion (205) and at least a second
portion (206);
a first piston (202) movable within the first portion (205) and separating
the first portion (205) into a first fluid chamber (205a) and a second fluid chamber (205b);
a second piston (203) movable within the second portion (206) and
separating the second portion (206) into a third fluid chamber (206a) and a fourth fluid chamber (206b); and
a valve system (300) selectively providing fluid communication between a
pressurized fluid source (211) and one or more of the fluid chambers (205a, 205b, 206a, 206b) and selectively providing fluid communication between the second fluid chamber (205b) and the third fluid chamber (206a).

2. The fluid operated actuation system (50) of claim 1, wherein the valve system (300) comprises a first valve (212) that selectively provides fluid communication between the pressurized fluid source (211) and the fourth fluid chamber (206b) in a first position and selectively provides fluid communication between the pressurized fluid source (211) and the first fluid chamber (205a) in a second position.

3. The fluid operated actuation system (50) of claim 2, wherein the valve system (300) further comprises a second valve (213) that selectively provides fluid communication between the first valve (212) and the third fluid chamber (206a).

4. The fluid operated actuation system (50) of claim 3, wherein the valve system (300) further comprises a third valve (214) that selectively provides fluid communication between the second and third fluid chambers (205b, 206a) in a first position and exhausts the second fluid chamber (205b) in a second position.

5. The fluid operated actuation system (50) of claim 1, further comprising a separating member (204) that separates the first and second portions (205, 206) of the housing (201).

6. The fluid operated actuation system (50) of claim 5, further comprising a first piston rod (207) coupled to the first piston (202) and extending through the separating member (204).

7. The fluid operated actuation system (50) of claim 6, wherein the first piston rod (207) is further coupled to the second piston (203).

8. The fluid operated actuation system (50) of claim 1, further comprising a second piston rod (208) coupled to the second piston (203) and extending through the housing (201).

9. A method of operating a fluid operated actuation system including a fluid operated actuator with a housing separated into a first portion and a second portion, a first piston movable in the first portion and separating the first portion into a first fluid chamber and a second fluid chamber, a second piston movable in the second portion and separating the second portion into a third fluid chamber and a fourth fluid chamber, and a first piston rod coupled to the first piston and extending from the first piston to contact the second piston, the method comprising steps of:
pressurizing the first fluid chamber;
exhausting the fourth fluid chamber, and
opening a fluid communication path between the second fluid chamber and the
third fluid chamber to actuate the first and second pistons from a first position to a second position with a first actuation force.

10. The method of claim 9, wherein the steps of pressurizing the first fluid chamber and exhausting the fourth fluid chamber comprises actuating a first valve from a first position to a second position.

11. The method of claim 9, wherein the step of opening the fluid communication path between the second and third fluid chambers comprises actuating a second valve and a third valve to a first position.

12. The method of claim 8, further comprising steps of:
closing the fluid communication path between the second and third fluid
chambers;
exhausting the second fluid chamber, and
pressurizing the third fluid chamber to actuate the first and second pistons from
the second position to a third position with a second actuation force.

13. The method of claim 12, wherein the steps of closing the fluid communication path between the second and third fluid chambers and exhausting the second fluid chamber comprises actuating a third valve to a second position.

14. The method of claim 12, wherein the step of pressurizing the third fluid chamber comprises actuating a second valve to a second position.
